# EUROPEAN PATENT SPECIFICATION

(11) **EP 3 912 595 B1**
(45) Date of publication and mention of the grant of the patent: **04.01.2023**
(21) Application number: 20020235.6
(22) Date of filing: 19.05.2020
(51) Int. Cl.: A61F 2/24

(54) **IMPLANT FOR IMPROVING COAPTATION OF AN ATRIOVENTRICULAR VALVE**
IMPLANTAT ZUR VERBESSERUNG DER CO-APTATION EINER ATRIOVENTRIKULÄREN KLAPPE
IMPLANT POUR AMÉLIORER LA COAPTATION D'UNE VALVE ATRIOVENTRICULAIRE

(43) Date of publication of application: 24.11.2021
(73) Proprietor: AVVie GmbH, 1090 Vienna (AT)
(72) Inventor: MOHL, Werner, 2571 Altenmarkt/Thennenberg (AT); SHAJI, Ashly, 1090 Vienna (AT); JARMAN, Jeremy, 1090 Vienna (AT)
(74) Representative: Keschmann, Marc

(56) References cited:
- WO-A2-2018/142217
- US-A1- 2014 067 048
- US-A1- 2019 175 344

## Description

The invention relates to an implant for improving coaptation of an atrioventricular valve.

Atrioventricular valves are membranous folds that prevent backflow from the ventricles of the human heart into the atrium during systole. The valves are anchored within the ventricular cavity by chordae tendineae, which prevent the valves from prolapsing into the atrium. The chordae tendineae are attached to papillary muscles that cause tension to better hold the valve. Together, the papillary muscles and the chordae tendineae are known as the subvalvular apparatus. The subvalvular apparatus prevents the valves from prolapsing into the atria when they close.

The human heart comprises two atrioventricular valves, the mitral valve and the tricuspid valve. The mitral valve allows the blood to flow from the left atrium into the left ventricle. The tricuspid valve is located between the right atrium and the right ventricle. The mitral valve has two leaflets that are each divided into several scallops: the anterior leaflet has three scallops (A1,A2,A3), the posterior leaflet has three scallops (P1,P2,P3). Further the mitral valve has an annulus. The tricuspid valve has three leaflets. Engagement of the corresponding surfaces of the leaflets against each other is decisive for providing proper closure of the valve to prevent that the blood flows in the wrong direction.

Native heart valves become dysfunctional for a variety of pathological causes. Failure of the leaflets to properly close during ventricular systole is known as malcoaptation and may allow the blood to flow backwards through the valve from the ventricle into the atrium. Malcoaptation is often caused by a dilatation of the annulus. Another cause of malcoaptation may be an excessive motion of the leaflet structures, which is due to local elongation or rupture of the chordae tendineae and results in a prolapse of parts of the leaflet into the atrium.

One of the main targets in clinical therapy of dysfunctional heart valves is hence to restore chordal function.

According to state of the art methods chordal function is often repaired by restoring one of the dysfunctional chords by fixing it with artificial chords, which are mainly made from PTFE or other polymers, to the muscular part of the left ventricle (i.e. the papillary muscle or the ventricular wall). A major disadvantage of said method is that the artificial chords are often oriented in a non-physiological way, which results in abnormal forces acting on the subvalvular apparatus, which causes reappearance of valvular dysfunction after a short period of time.

Hence, there is the need to provide improved implants for improving coaptation of an atrioventricular valve, which restore chordal function and effectively prevent prolapse of the native leaflet into the atrium. Further, said improved implants should effectively withstand the forces prevailing in the heart.

An attempt in providing such an improved implant is disclosed in WO2017/115123A1. The implant of WO2017/115123A1 comprises a support structure with an upper support element to be arranged on and fixed to the superior surface of the annulus and a lower support element to be arranged on and fixed to the inferior surface of the annulus thereby clamping a section of the annulus in between. Further, the implant comprises an artificial leaflet fixed to the support structure and a holding arm being fixed to the lower support structure, which holds the artificial leaflet from below and thereby prevents prolapse of the artificial leaflet. However, the holding arm of the implant of WO2017/115123A1 projects into the left ventricle thereby constituting an additional artificial structure in the ventricle. A further disadvantage of the implant of WO2017/115123A1 is that the holding arm, which is attached to the lower support structure, is swinging up and down together with the movement of the leaflet, which swinging motion may subject the holding arm to oscillations. Such oscillations cause a periodic bending of the elastic arm, which may negatively affect the durability of the implanted device and therefore the function of holding back the artificial leaflet to prevent a prolapse.

WO2018/142217A2 discloses an implant with a backbone element, which is bendable between a first state of bending and a second state of bending, whereby limiting means are provided for limiting the degree of bending.

Therefore, it is an object of the instant invention to provide an improved and stable implant, which does without arranging an additional artificial structure, such as a holding arm at the lower side of the leaflet and effectively improves coaptation and prevents prolapse of the dysfunctional leaflet into the atrium.

Throughout the whole description the term "dysfunctional leaflet" corresponds to the term "at least one of the first and second native leaflets". The term "superior" corresponds to the term "upper" and the term "inferior" corresponds to the term "lower". The term "side" corresponds to the term "surface".

In order to achieve the above object the invention provides for an implant according to claim 1. The implant comprises an artificial leaflet configured to replace or support at least one of the first and second native leaflets, fixing means for fixing the implant to the annulus or to the at least one of the first and second native leaflets and retention means for preventing prolapse of the artificial leaflet, whereby the retention means comprise or consist of a backbone element extending along the artificial leaflet and towards a distal end of the artificial leaflet and being connected to the artificial leaflet at at least two attachment points, wherein the backbone element is configured to be bendable between a first state of bending and a second state of bending and wherein limiting means are provided for limiting the degree of bending.

The fixing means of the implant may be configured to fix the artificial leaflet onto the upper surface of the annulus or of the dysfunctional leaflet, i.e., the surface which faces towards the left atrium. Preferably, the fixing means may be fixed on the P2 segment of the posterior native leaflet of the mitral valve.

By fixing the artificial leaflet to the upper surface of the annulus, the artificial leaflet may at least partly cover the upper surface of the dysfunctional leaflet, i.e., the surface of the dysfunctional leaflet, which faces towards the atrium.

To effectively prevent prolapse of the artificial leaflet as well as of the dysfunctional leaflet into the atrium, the implant according to the invention comprises retention means, which either comprise or consist of a backbone element, which extends along the artificial leaflet and towards the distal end of the artificial leaflet. The backbone element may be designed as an elongate element that may be connected to the fixing means in its proximal region and extends towards the distal end of the artificial leaflet. By being connected to the artificial leaflet at at least two attachment points, the backbone element, in its function as retention means, restricts the movement of the artificial leaflet in accordance with the current shape, form or curvature defined by the backbone element. Preferably, the backbone element may be connected to the artificial leaflet over its entire length, so that the flexible artificial leaflet fully adjusts to the shape, form or curvature of the backbone element. For example, the backbone element, may be integrated into the artificial leaflet. Alternatively, the backbone element may be arranged on and connected to the upper and/or the lower side of the artificial leaflet.

The backbone element, as is the artificial leaflet, may be arranged on the upper side of the dysfunctional leaflet.

The backbone element is configured to be bendable between a first state of bending, which corresponds to an open valve position, in which the blood is allowed to flow from the left atrium into the left ventricle, and a second state of bending, which corresponds to a closed valve position, in which backflow of blood from the left ventricle into the left atrium is prevented.

The limited degree of bending of the backbone element, which is provided according to the invention, provides for proper valve closure and forms a proper coaptation plane with the unaffected leaflet of the mitral valve, because the upwards movement of the retention means is blocked when the artificial leaflet reaches the closed valve position, and prevents a prolapse of the artificial leaflet into the atrium, because further movement towards the atrium is effectively blocked.

The implant according to the invention hence is able to restore chordal function of a dysfunctional native leaflet, by covering the same with the artificial leaflet according to the invention, whereby the function is restored mainly by the backbone element without the need of holding the dysfunctional or artificial leaflet from below by arranging an additional structure, such as a holding element that protrudes into the ventricular cavity.

According to the invention, the backbone element comprises a plurality of segments, pivotably connected to each other. To limit the degree of bending of the individual segments of the backbone element, the limiting means of the implant comprise an abutment element on each segment for limiting the pivoting angle of one segment relative to an adjacent segment. Preferably, the abutment element is rigidly fixed to the segment or integrally formed with the segment in a single piece of material. By limiting the pivoting angle, the movement of the backbone element is controlled and prolapse of the artificial leaflet into the atrium may be effectively prevented, while simultaneously providing for proper coaptation of the valve. Preferably, the abutment element comprises and abutment surface that protrudes from its associated segment towards an adjacent segment. The adjacent segment comprises a counter surface that abuts with the abutment surface, if a defined pivoting angle is reached.

Preferably, the limiting means are configured such that the backbone element, in at least a partial section thereof, has a smaller radius of curvature in its second state of bending than in its first sate of bending.

To better align with the anatomical curvature of the dysfunctional native leaflet in the closed as well as the open valve position, the degree of bending of the backbone element is preferably chosen such that it is bendable to a higher degree in its middle section than at its proximal and distal ends. The variation in its maximum possible bendability over the length of the backbone element is preferably achieved by varying the limitation of the pivoting angles of the connected individual segments imparted by the limiting means. In a preferred embodiment, wherein the limiting means comprise an abutment element, the angle at which the abutment surface of the abutment element extends from its associated segment determines the maximum possible pivoting angle.

The implant acts like a swing that swings back and forth, whereby the forth movement is stopped when the abutment elements on each of the segments limit the pivoting angle relative to the adjacent segments.

The segments of the backbone element are preferably made from bio ceramics.

To provide additional support and to stabilize the implant when being fixed to the heart tissue, the implant preferably comprises a resting element, which is fixed to the proximal end of the backbone element and configured to rest against the wall of the atrium adjacent the valve. The resting element provides for a high level of stability, because it is configured to rest against the wall of the atrium. Thereby the implant may effectively resist to the tilting forces acting upon it by the dynamic pressure changes prevailing within the ventricle.

The resting element is preferably half-ring or u-shaped or has a wing-like or cushion-like structure.

If stress of varying intensity caused by the blood flow and the blood pressure acts on the valve, the implant tends to swing back and forth. In order to reduce such swinging movement, the resting element preferably comprises a support element being aligned with the backbone element and configured to rest against the wall of the atrium adjacent the valve. The support element is configured to be in tight contact with the wall of the atrium during the swinging movement. Preferably, the support element comprises or consist of a curved element, the curvature of which is preferably adapted to the curvature of the wall of the atrium adjacent the valve. Thereby the stabilization properties of the implant are enhanced. In this way, the tilting forces acting on the backbone element can be better resisted by the support element, which adjoins the backbone element, i.e. prolongs the backbone element in its axial direction.

The resting element preferably is shaped as to correspond to the curvature of the left ventricle.

Preferably the resting element is made of a shape-memory alloy, such as, e.g. nitinol.

Preferably, the artificial leaflet comprises a mesh or a flexible net or a plurality of flexible wires or yarns or a fabric or a sheet, preferably a PTFE sheet or matrix, which cover an area that substantially corresponds to the area of the superior surface of the native leaflet. Thereby the anatomical structure of the dysfunctional leaflet is mimicked and its function might be restored even more effectively.

More generally, the artificial leaflet may be any flexible, preferably planar, structure that can at least partially cover the dysfunctional leaflet so as to control the opening and closing movement of the dysfunctional leaflet. The artificial leaflet may be a continuous structure or a discontinuous structure, i.e. a structure that has openings, such as a net. The artificial leaflet may also be designed as a plurality of wires that are connected to the backbone element and stabilize the dysfunctional leaflet.

If the artificial leaflet comprises a mesh, the shape of the mesh preferably varies depending on the position of the prolapse on the leaflet. If the prolapse is on the P2 segment of the posterior leaflet of the mitral valve, the mesh preferably has a convex shape. If the prolapse is between the P2 and the P3 segment of the posterior leaflet of the mitral valve, the mesh preferably has a concave shape. If the prolapse is on the anterior leaflet of the mitral valve, the mesh preferably has a convex shape.

To provide stability and to ensure that the artificial leaflet remains spread out over the entire dysfunctional leaflet, the artificial leaflet preferably comprises a wire frame extending along a periphery of the artificial leaflet, which is preferably connected to at least two ribs extending perpendicularly from the backbone element.

The wire frame and the ribs are preferably made of nitinol.

To facilitate tissue ingrowth of the implant, the side of the artificial leaflet, which faces towards the native leaflet, preferably has a porous surface. The side of the artificial leaflet, which faces towards the atrium, preferably has an anticoagulant surface thereby preventing coagulation of blood on the upper surfaces of the artificial leaflet.

To avoid injury of the heart tissue, which surrounds the implant, the backbone element is preferably covered by a sheath. Said sheath also enables for the use of a transseptal catheter during implantation.

To ensure alignment of the artificial leaflet with the dysfunctional leaflet and to provide a proper connection between those two, a distal end section of the backbone element preferably comprises a u-bend so as to clamp a distal region of the dysfunctional native leaflet between segments of the backbone element.

In order to achieve a sufficient clamping force, spring means are preferably arranged between segments arranged in the distal end section of the backbone element, in order to resiliently press segments of the backbone element against the distal region of the dysfunctional leaflet.

To cover the dysfunctional leaflet on its upper as well as its lower side, the backbone element preferably comprises a first section adapted to be arranged on the upper side of the dysfunctional leaflet and a second section adapted to be arranged on the lower side of the dysfunctional leaflet, wherein the first and the second section are connected with each other by said u-bend. Preferably, the second section extends from the u-bend to the lower side of the annulus. In this way, full enclosure and full coverage of the dysfunctional leaflet is achieved. By said preferred embodiment the dysfunctional leaflet is squeezed by the backbone element from its upper as well as its lower side without leaving any space between the backbone element and the dysfunctional native leaflet, whereby effective stabilization of the implant is achieved without the need of arranging an additional ventricular component, such as a holding arm that protrudes into the ventricular cavity.

To provide a stable connection between the implant and the valve, the fixing means is preferably arranged in the proximal end region of the artificial leaflet, and is preferably aligned with the backbone element, and comprises a central needle unit configured to penetrate the annulus or the at least one of the first and second native leaflets in a penetration region, and a stabilization platform having an abutment surface for abutting the penetration region, whereby the central needle unit and the stabilization platform are movably arranged in a housing in an axial direction of the needle.

Preferably, the penetration region may be arranged in the region of the posterior leaflet of the mitral valve that is having a prolapse. In case that the middle region of the mitral valve is prolapsing the penetration region may be arranged at the P2 region of the posterior leaflet of the mitral valve. In case the prolapsing segment is in the indentation of the P1 segment or the P3 segment, the penetration site may preferably be arranged at the center of the prolapsing segment.

In case of an anterior leaflet prolapse the penetration site is preferably at the A2 segment.

To independently move the needle unit and the stabilization platform, the fixing means preferably comprises first spring means acting on the needle unit for displacing the needle unit relative to the housing and second spring means acting on the stabilization platform for displacing the stabilization platform relative to the housing.

For subsequent deployment of its individual components, the fixing means further comprises a first releasable locking means to hold the central needle unit in a spring-loaded position and a second releasable locking means to hold the stabilization platform in a spring-loaded position.

Preferably, the central needle unit comprises anchoring elements on its distal end, said anchoring elements being movable relative to the needle unit between a first position, wherein the anchoring elements are positioned against a needle shaft, and a second position, wherein the anchoring elements are tilted away from the needle shaft.

The fixing means is preferably deployed in two subsequent steps.

In a first step the first locking means is released, whereby the first spring means causes the central needle unit to move towards the upper surface of the native leaflet and subsequently penetrate the leaflet. During said movement the tip of the central needle unit is pushed out of the needle guide and the anchoring elements of the central needle unit deploy. Following the penetration of the leaflet, the deployed anchoring elements are located on the lower side of the native leaflet after said first step.

In a second step the second locking means of the fixing means is released, whereby the second spring means causes the abutment surface of the stabilization platform to move towards the upper surface of the native leaflet and hence also towards the deployed anchoring elements on the lower surface of the native leaflet. By the movement of the stabilization platform, the upper surface of the leaflet is pushed towards the anchoring elements, whereby the distance between the abutment surface of the stabilization platform and the anchoring elements is reduced so as to clamp the native leaflet between the abutment surface of the fixing means and the anchoring elements. Said clamping results in that the anchoring elements are pressed into the native leaflet from below in order to achieve a stable connection of the deployed anchoring elements to the tissue on the lower side of the native leaflet.

To protect the surrounding tissue the anchoring elements are preferably covered by a folded protecting means, which covers the anchoring elements when the tip of the central needle unit is in the needle guide. After the tip of the central needle unit has left the needle guide and penetrated the native leaflet, the anchoring means deploy and the protecting means unfold on the lower surface of the native leaflet, whereby the distal end of the anchoring elements is covered by the protecting means.

The implant according to the invention may be fixed to the posterior leaflet of the mitral valve or the anterior leaflet of the mitral valve.

The implant of the invention is preferably designed for being delivered to the heart transatrially, transseptally or transfemorally. Therefore, the implant may be configured for arrangement in a tubular delivery device, such as a catheter.

Preferably, the artificial leaflet and the resting element are deployable from a first position, in which the artificial leaflet and the resting element are folded for being arranged within a tubular housing of a delivery device, into a second position, in which the artificial leaflet and the resting element are deployed. Thereby, the implant can be easily deployed to the heart by minimal invasive surgery or endovascular approaches.

In particular, the tubular housing is preferably advanced into the heart by means of a catheter or a deployment instrument transatrially, transseptally or transfemorally.

To not only restore chordal function, but to also attend to the second main cause of malcoaptation, i.e. dilatation of the annulus, the implant preferably further comprises an annuloplasty device for reducing the circumference of the annulus, which comprises at least a first and a second anchor unit, preferably a plurality of anchor units, that are deployable to the mitral valve by means of a vascular delivery device, such as a catheter, and positionable in a row along the annulus, comprising anchor means arranged on each of the first and second anchor units, whereby the anchor units are arranged to be movable towards one another, so that the anchor means engage with the annulus thereby pulling the annulus together when the first and second anchor units are moved.

The annuloplasty device is preferably connected to the proximal end of the implant, i.e. fixed to the implant where the fixing means is arranged.

The annuloplasty device is preferably built as a multipart telescopic tube with a wire going through its center and being connected to each of the parts of the telescopic tube as well as to the central needle unit.

Each part of the telescopic tube constitutes an individual anchor unit and is positioned in a row along the annulus.

When the circumference of the annulus is to be reduced, the central needle unit is rotated about its central axis, whereby the wire is coiled around the central needle unit thereby reducing wire length, whereby the anchor units, which are also attached to the wire are retracted towards the center of the annuloplasty device.

The movement of the anchor units results in the penetration of the needle units into the tissue, whereby the tissue is grabbed and the circumference of the annulus is reduced by 20-30%, because the tissue between the needle units is cinched.

In the following the present invention will be described by reference to some exemplary embodiments.

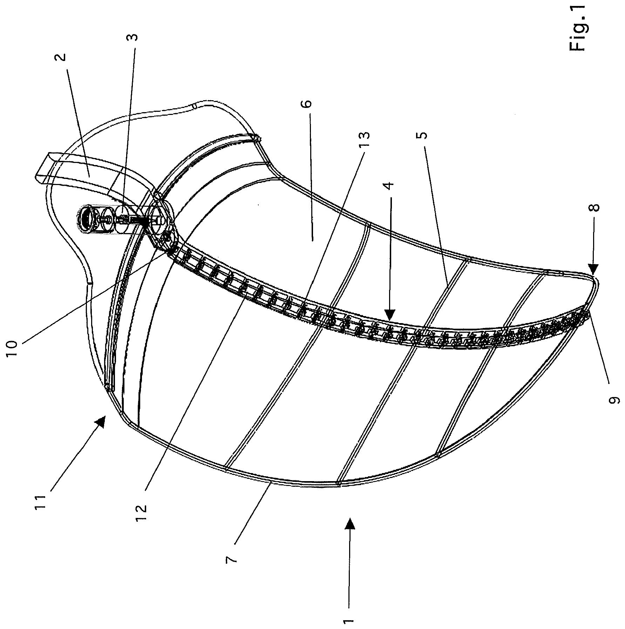

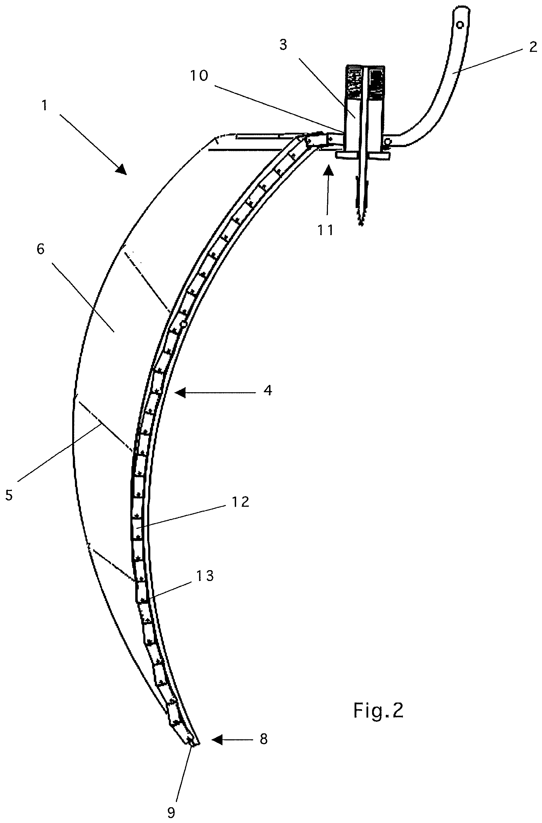

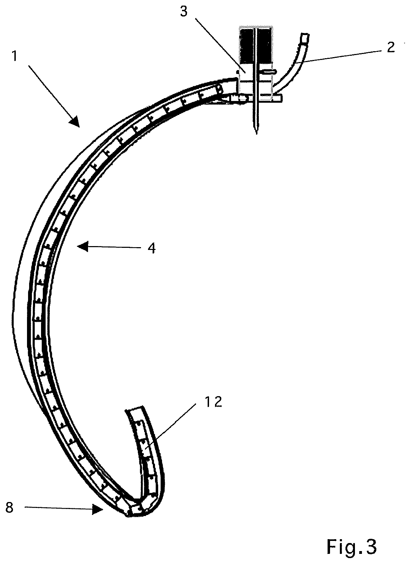

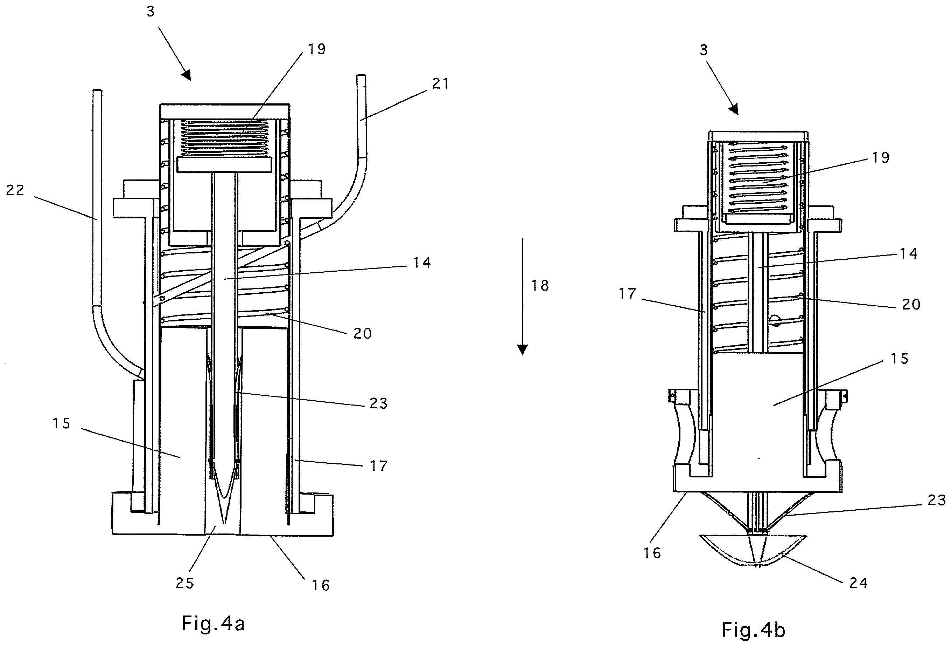

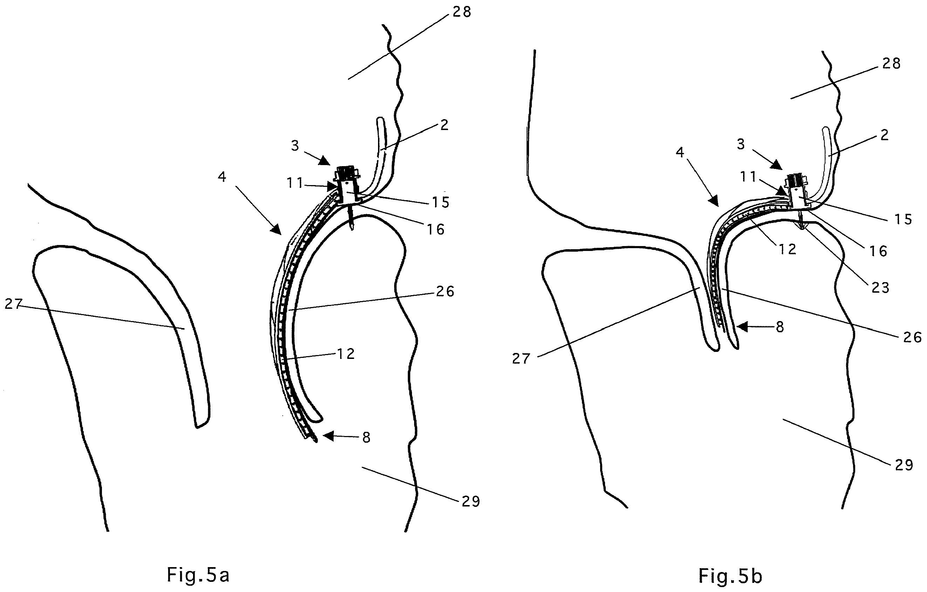

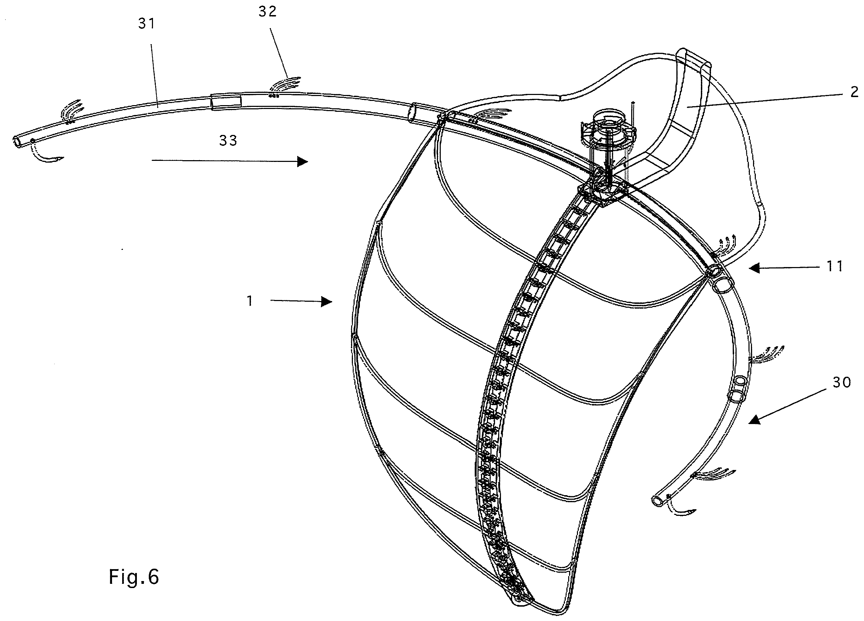

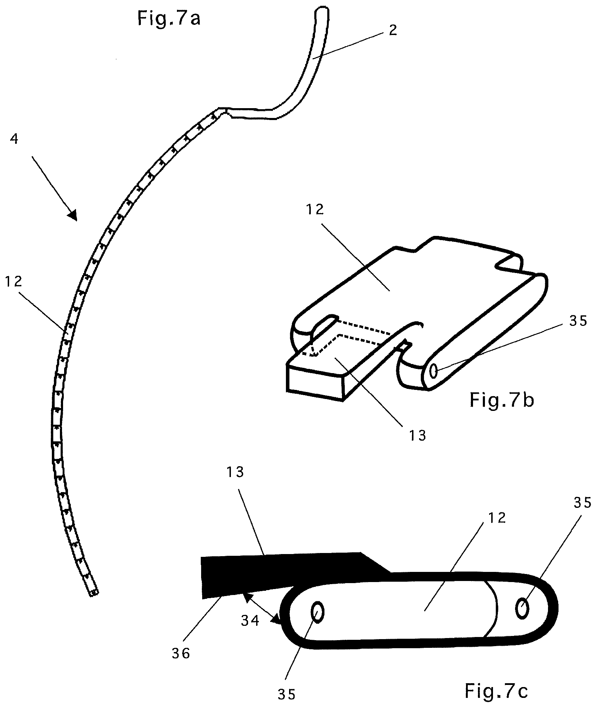

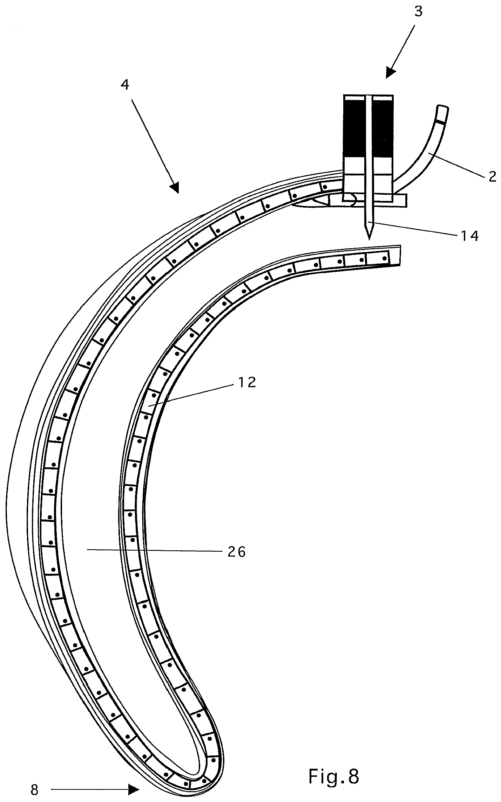

Fig.1 is an isometric view of a first embodiment of the implant according to the invention, Fig.2 is a cross sectional view of the first embodiment of the implant as depicted in Fig.1, Fig.3 is a cross sectional view of a second embodiment of the implant according to the invention, Fig.4a shows a cross sectional view of the fixing means of the implant according to the invention before deployment, Fig.4b shows a cross sectional view of the fixing means of Fig.4a after deployment, Fig.5a shows a cross sectional view of the implant as depicted in Fig.1 and Fig.2 when being fixed to the posterior leaflet of the mitral valve during atrial systole, Fig.5b shows a cross sectional view of the implant as depicted in Fig.5a during ventricular systole, Fig.6 shows an isometric view of a third embodiment of the implant according to the invention when being combined with an annuloplasty device, Fig.7a shows a sectional view of the backbone element according to a fist embodiment of the invention, Fig.7b shows an isometric view of one segment of the backbone element as depicted in Fig.7a, Fig. 7c shows a sectional view of the segment as depicted in Fig.7b and Fig.8 shows a cross-sectional view of a fourth embodiment of the invention.

Fig.1 and Fig.2 show a first embodiment of the implant according to the invention. The implant comprises an artificial leaflet 1, a support element 2, fixing means 3 and retention means. The artificial leaflet comprises a mesh or a sheet 6. The retention means comprise a backbone element 4. Further, a plurality of ribs 5 extending perpendicularly from the backbone element 4, and a wire frame 7 extending along a periphery of the artificial leaflet 1 are provided. The backbone element 4 may be perforated at certain intervals for arranging the ribs 5.

The backbone element 4 extends along the artificial leaflet 1 and towards its distal end 8 and is connected to the artificial leaflet 1 at least at attachment points 9 and 10. Attachment point 9 is located on the distal end 8 of the artificial leaflet 1 and attachment point 10 is located on the proximal end 11 of the artificial leaflet 1.

The backbone element 4 comprises a plurality of segments 12, which are pivotably connected to each other, whereby abutment elements 13 are provided on the distal ends of each of the segments 12 for limiting the pivoting angle of one segment relative to the adjacent segment.

The backbone element 4 and the segments 12 thereof are shown in more detail in Fig. 7a-c. The segments 12 each comprise an abutment element 13 that extends from one end of the segment 12. The segment comprises a pivoting axis 35 on both ends thereof for pivotably being connected to an adjacent segment 12. The abutment element 13 comprises an abutment surface 36 that defines the maximal pivoting angle relative to the adjacent segment 12. In other words, the angular extension or inclination 34 of the abutment surface 36 limits the pivoting angle of one segment relative to the adjacent segment 12.

Fig.3 shows a cross sectional view of a second embodiment of the implant according to the invention, which essentially corresponds to the first embodiment as depicted in Fig.1 and Fig.2, but comprises a u-bend in the distal end section 8 of the backbone element 4, which enables clamping of a distal region of the dysfunctional native leaflet between the segments 12 of the distal end section 8 of the backbone element 4.

Fig.4a shows a cross sectional view of the fixing means of the implant according to the invention before deployment, and Fig.4b shows a cross sectional view of the fixing means of Fig.4a after deployment.

Fig.4a shows that the fixing means 3 comprises a central needle unit 14 and a stabilization platform 15 having an abutment surface 16. The central needle unit 14 and the stabilization platform 15 are arranged in a housing 17 and both moveable in an axial direction according to arrow 18. For causing said axial movement, the fixing means 3 comprises first spring means 19 acting on the needle unit 14 and second spring means 20 acting on the stabilization platform 15.

The fixing means 3 further comprises a first releasable locking means 21 to hold the central needle unit 14 in the spring-loaded position, whereby the spring-load is exerted by the first spring means 19, and a second releasable locking means 22 to hold the stabilization platform 15 in a spring-loaded position, whereby the spring-load is exerted by the second spring means 20.

Further, the central needle unit 14 comprises anchoring elements 23 on its distal end, which are covered by a protecting means 24, which covers the anchoring elements 23 when the tip of the central needle unit 14 is in the needle guide 25.

When the first locking means 21 is released by being pulled out of the housing 17, the first spring means 19 causes the central needle unit 14 to move according to arrow 18. During said movement the central needle unit 14 is pushed out of the needle guide 25 and the anchoring elements 23 as well as the protecting means 24 of the central needle unit 14 deploy as shown in Fig.4b.

The anchoring elements 23 and the protecting means 24 are movable relative to the central needle unit 14 between a first, undeployed position as shown in Fig.4a, in which the anchoring elements 23 and the protecting means 24 are positioned against the needle shaft 25, and a second, deployed position as shown in Fig.4b, in which the anchoring elements 23 and the protecting means 24 are tilted away from the needle shaft 25.

When the second locking means 22 is released by being pulled out of the housing 17, the second spring means 20 causes the stabilization platform 15 to move according to arrow 18 towards the deployed anchoring elements 23 so as to clamp the leaflet tissue that is arranged between the abutment surface 16 and the anchoring elements 23.

Fig.5a shows a cross sectional view of the implant according to the first embodiment of the invention as depicted in Fig.1 and Fig.2 being fixed to the posterior native leaflet 26 of the mitral valve, wherein the mitral valve is in its open position. Therefore the posterior leaflet 26 and the anterior leaflet 27 are in an open position, which allows blood flow from the left atrium 28 into the left ventricle 29.

The fixing means 3 is fixed to the annular region of the posterior leaflet 26 and the support element 2, which is aligned with the backbone element 4 of the artificial leaflet 1 rests against the wall of the left atrium 28 in order to resist to the tilting forces acting upon the artificial leaflet 1, which are caused by the dynamic pressure changes prevailing within the left ventricle 29.

Fig.5b shows a cross sectional view of the implant as depicted in Fig.5a during ventricular systole, i.e. when the posterior leaflet 26 and the anterior leaflet 27 are in a closed position, whereby the blood flow from the left atrium 28 into the left ventricle 29 is blocked. The degree of bending of the individual segments 12 of the backbone element 4 is higher in its middle section and lower at its proximal end 11 and at its distal end 8, whereby proper valve closure is allowed and prolapse of the artificial leaflet is prevented.

When being fixed to the annulus of the posterior leaflet 26 the central needle unit 14 has penetrated the annulus, whereby the anchoring elements 23 are arranged facing the lower surface of the posterior leaflet 26 and the abutment surface 15 of the stabilization platform 15 is arranged on the upper surface of the posterior leaflet 26 and abuts the penetration region to press the anchoring elements 23 towards the abutment surface 15 and to enable an anchoring effect on the lower side of the leaflet 26.

Fig.6 shows an isometric view of a third embodiment of the implant according to the invention when being combined with an annuloplasty device 30.

The annuloplasty device 30 and the artificial leaflet 1 are connected in the proximal region 11 of the artificial leaflet 1. The annuloplasty device 30 comprises a plurality of anchor units 31 with anchor means 32 being arranged on each of said anchor units 31.

When the combined implant as depicted in Fig.6 is fixed to the posterior leaflet 26 of the mitral valve the anchor units 31 are positioned in a row along the curvature of the annulus, whereby the anchor means 32 engage with the annulus when the anchor units 31 are moved according to arrow 33 thereby pulling the annulus together.

Fig.8 shows a cross-sectional view of a fourth embodiment according to the invention, in which the distal end section 8 of the backbone element 4 comprises a u-bend, which encloses the distal region of the dysfunctional native leaflet 26 and the backbone element 4 further extends towards the lower side of the annulus, i.e. towards the region where the central needle unit 14 has penetrated the native leaflet 26. Thereby full coverage of the dysfunctional leaflet 26 is achieved, i.e. the leaflet 26 is covered by the backbone element 4 on its upper as well as its lower surface.

## Claims

1. An implant for improving coaptation of an atrioventricular valve in a heart, the atrioventricular valve having a native first leaflet, a native second leaflet and optionally an annulus adjacent a wall of an atrium of the heart, the implant comprising an artificial leaflet (1) configured to replace or support at least one of the first and second native leaflets, fixing means (3) for fixing the implant to the annulus or to the at least one of the first and second native leaflets and retention means for preventing prolapse of the artificial leaflet, wherein the retention means comprise or consist of a backbone element (4) extending along the artificial leaflet (1) and towards a distal end (8) of the artificial leaflet (1) and being connected to the artificial leaflet (1) at at least two attachment points (9,10), wherein the backbone element (4) is configured to be bendable between a first state of bending and a second state of bending, wherein limiting means are provided for limiting the degree of bending, and wherein the backbone element (4) comprises a plurality of segments (12), pivotably connected to each other, **characterized in that** the limiting means comprise an abutment element (13) on each segment (12) for limiting the pivoting angle of one segment relative to an adjacent segment.

2. The implant according to claim 1, **characterized in that** the implant comprises a resting element being fixed to the proximal end of the backbone element (4) and configured to rest against the wall of the atrium adjacent the valve.

3. The implant according to claim 2, **characterized in that** the resting element comprises a support element (2) being aligned with the backbone element (4) and configured to rest against the wall of the atrium adjacent the valve.

4. The implant according to any one of claims 1,2 or 3, **characterized in that** the artificial leaflet (1) comprises a mesh (6) or a flexible net or a plurality of flexible wires or yarns or a fabric or a sheet, preferably a PTFE sheet or matrix.

5. The implant according to any one of claims 1 to 4, **characterized in that** the artificial leaflet (1) comprises a wire frame (7) extending along a periphery of the artificial leaflet (1), which is preferably connected to at least two ribs (5) extending perpendicularly from the backbone element (4).

6. The implant according to any one of claims 1 to 5, **characterized in that** the side of the artificial leaflet (1), which faces towards the atrium, has an anticoagulant surface and the side of the artificial leaflet (1), which faces towards the first or second native leaflet, has a porous surface.

7. The implant according to any one of claims 1 to 6, **characterized in that** the backbone element (4) is covered by a sheath.

8. The implant according to any one of claims 1 to 7, **characterized in that** a distal end section (8) of the backbone element (4) comprises a u-bend so as to clamp a distal region of the first or second native leaflet between segments of the backbone element (4).

9. The implant according to claim 8, **characterized in that** spring means are arranged between segments (12) arranged in the distal end section (8) of the backbone element (4), in order to resiliently press segments (12) of the backbone element (4) against the distal region of the first or second native leaflet.

10. The implant according to any one of claims 1 to 9, **characterized in that** the fixing means (3) is arranged in the proximal end region (11) of the artificial leaflet (1), and is preferably aligned with the backbone element (4), and comprises a central needle unit (14) configured to penetrate the annulus or the at least one of the first and second native leaflets in a penetration region, and a stabilization platform (15) having an abutment surface (16) for abutting the penetration region, whereby the central needle unit (14) and the stabilization platform (15) are movably arranged in a housing (17) in an axial direction of the needle.

11. The implant according to claim 10, **characterized in that** the fixing means (3) comprises first spring means (19) acting on the needle unit (14) for displacing the needle unit (14) relative to the housing (17) and second spring means (20) acting on the stabilization platform (15) for displacing the stabilization platform (15) relative to the housing (17).

12. The implant according to claim 11, **characterized in that** the fixing means (3) further comprises a first releasable locking means (21) to hold the central needle unit (14) in a spring-loaded position and a second releasable locking means (22) to hold the stabilization platform (15) in a spring-loaded position.

13. The implant according to claim 10, 11 or 12, **characterized in that** the central needle unit (14) comprises anchoring elements (23) on its distal end, said anchoring elements (23) being movable relative to the central needle unit (14) between a first position, wherein the anchoring elements (23) are positioned against a needle shaft (25), and a second position, wherein the anchoring elements (23) are tilted away from the needle shaft (25).

14. The implant according to any one of claims 2 to 13, **characterized in that** the artificial leaflet (1) and the resting element are deployable from a first position, in which the artificial leaflet (1) and the resting element are folded for being arranged within a tubular housing of a delivery device, into a second position, in which the artificial leaflet (1) and the resting element are deployed.

15. The implant according to any one of claims 1 to 14, **characterized in that** the implant further comprises an annuloplasty device (30) comprising at least a first and a second anchor unit (31), preferably a plurality of anchor units, that are deployable to the mitral valve by means of a vascular delivery device, such as a catheter, and positionable in a row along the annulus, comprising anchor means (32) arranged on each of the first and second anchor units (31), whereby the anchor units (31) are arranged to be movable towards one another, so that the anchor means (32) engage with the annulus thereby pulling the annulus together when the first and second anchor units (31) are moved.

## Patentansprüche

1. Implantat zur Verbesserung der Koaptation einer atrioventrikulären Klappe in einem Herzen, wobei die atrioventrikuläre Klappe ein natives erstes Segel, ein natives zweites Segel und optional einen Annulus angrenzend an eine Wand eines Vorhofs des Herzens aufweist, wobei das Implantat ein künstliches Segel (1), das so konfiguriert ist, dass es mindestens eines der ersten und zweiten nativen Segel ersetzt oder stützt, Befestigungsmittel (3) zum Befestigen des Implantats am Annulus oder an dem mindestens einen der ersten und zweiten nativen Segel und Rückhaltemittel zum Verhindern eines Prolapses des künstlichen Segels umfasst, wobei die Rückhaltemittel ein Hauptstrangelement (4) umfassen oder daraus bestehen, das sich entlang des künstlichen Segels (1) und in Richtung eines distalen Endes (8) des künstlichen Segels (1) erstreckt und mit dem künstlichen Segel (1) an mindestens zwei Befestigungspunkten (9, 10) verbunden ist, wobei das Hauptstrangelement (4) so konfiguriert ist, dass es zwischen einem ersten Biegezustand und einem zweiten Biegezustand biegbar ist, wobei Begrenzungsmittel vorgesehen sind, um den Grad der Biegung zu begrenzen, und wobei das Hauptstrangelement (4) eine Vielzahl von Segmenten (12) umfasst, die schwenkbar miteinander verbunden sind, **dadurch gekennzeichnet, dass** die Begrenzungsmittel ein Anschlagelement (13) an jedem Segment (12) umfassen, um den Schwenkwinkel eines Segments relativ zu einem benachbarten Segment zu begrenzen.

2. Implantat nach Anspruch 1, **dadurch gekennzeichnet, dass** das Implantat ein Auflageelement umfasst, das am proximalen Ende des Hauptstrangelements (4) befestigt und so konfiguriert ist, dass es an der Wand des Vorhofs neben der Klappe anliegt.

3. Implantat nach Anspruch 2, **dadurch gekennzeichnet, dass** das Auflageelement ein Stützelement (2) umfasst, das mit dem Hauptstrangelement (4) ausgerichtet ist und so konfiguriert ist, dass es an der Wand des Vorhofs neben der Klappe anliegt.

4. Implantat nach einem der Ansprüche 1, 2 oder 3, **dadurch gekennzeichnet, dass** das künstliche Segel (1) ein Gitter (6) oder ein flexibles Netz oder eine Vielzahl von flexiblen Drähten oder Garnen oder ein Gewebe oder eine Folie, vorzugsweise eine PTFE-Folie oder -Matrix, umfasst.

5. Implantat nach einem der Ansprüche 1 bis 4, **dadurch gekennzeichnet, dass** das künstliche Segel (1) einen sich entlang eines Umfangs des künstlichen Segels (1) erstreckenden Drahtrahmen (7) umfasst, der vorzugsweise mit mindestens zwei Rippen (5) verbunden ist, die sich senkrecht von dem Hauptstrangelement (4) erstrecken.

6. Implantat nach einem der Ansprüche 1 bis 5, **dadurch gekennzeichnet, dass** die dem Vorhof zugewandte Seite des künstlichen Segels (1) eine gerinnungshemmende Oberfläche und die dem ersten oder zweiten nativen Segel zugewandte Seite des künstlichen Segels (1) eine poröse Oberfläche aufweist.

7. Implantat nach einem der Ansprüche 1 bis 6, **dadurch gekennzeichnet, dass** das Hauptstrangelement (4) von einem Mantel umhüllt ist.

8. Implantat nach einem der Ansprüche 1 bis 7, **dadurch gekennzeichnet, dass** ein distaler Endabschnitt (8) des Hauptstrangelements (4) eine U-Biegung aufweist, um einen distalen Bereich des ersten oder zweiten nativen Segels zwischen Segmenten des Hauptstrangelements (4) einzuklemmen.

9. Implantat nach Anspruch 8, **dadurch gekennzeichnet, dass** zwischen Segmenten (12), die im distalen Endabschnitt (8) des Hauptstrangelements (4) angeordnet sind, Federmittel angeordnet sind, um Segmente (12) des Hauptstrangelements (4) elastisch gegen den distalen Bereich des ersten oder zweiten nativen Segels zu drücken.

10. Implantat nach einem der Ansprüche 1 bis 9, **dadurch gekennzeichnet, dass** das Befestigungsmittel (3) im proximalen Endbereich (11) des künstlichen Segels (1) angeordnet und vorzugsweise mit dem Hauptstrangelement (4) ausgerichtet ist und eine zentrale Nadeleinheit (14) umfasst, die so konfiguriert ist, dass sie den Annulus oder das mindestens eine der ersten und zweiten nativen Segel in einem Penetrationsbereich durchsticht, und eine Stabilisierungsplattform (15) mit einer Anlagefläche (16) zum Anlegen an den Penetrationsbereich, wobei die zentrale Nadeleinheit (14) und die Stabilisierungsplattform (15) in einem Gehäuse (17) in einer axialen Richtung der Nadel beweglich angeordnet sind.

11. Implantat nach Anspruch 10, **dadurch gekennzeichnet, dass** die Befestigungsmittel (3) erste Federmittel (19), die auf die Nadeleinheit (14) wirken, um die Nadeleinheit (14) relativ zum Gehäuse (17) zu verschieben, und zweite Federmittel (20), die auf die Stabilisierungsplattform (15) wirken, um die Stabilisierungsplattform (15) relativ zum Gehäuse (17) zu verschieben, umfassen.

12. Implantat nach Anspruch 11, **dadurch gekennzeichnet, dass** das Befestigungsmittel (3) weiterhin ein erstes lösbares Verriegelungsmittel (21) umfasst, um die zentrale Nadeleinheit (14) in einer federbelasteten Position zu halten, und ein zweites lösbares Verriegelungsmittel (22), um die Stabilisierungsplattform (15) in einer federbelasteten Position zu halten.

13. Implantat nach Anspruch 10, 11 oder 12, **dadurch gekennzeichnet, dass** die zentrale Nadeleinheit (14) an ihrem distalen Ende Verankerungselemente (23) aufweist, wobei die Verankerungselemente (23) relativ zur zentralen Nadeleinheit (14) zwischen einer ersten Position, in der die Verankerungselemente (23) gegen einen Nadelschaft (25) positioniert sind, und einer zweiten Position, in der die Verankerungselemente (23) vom Nadelschaft (25) weggekippt sind, beweglich sind.

14. Implantat nach einem der Ansprüche 2 bis 13, **dadurch gekennzeichnet, dass** das künstliche Segel (1) und das Auflageelement aus einer ersten Position, in der das künstliche Segel (1) und das Auflageelement gefaltet sind, um in einem röhrenförmigen Gehäuse einer Abgabevorrichtung angeordnet zu werden, in eine zweite Position gebracht werden können, in der das künstliche Segel (1) und das Auflageelement entfaltet sind.

15. Implantat nach einem der Ansprüche 1 bis 14, **dadurch gekennzeichnet, dass** das Implantat ferner eine Annuloplastievorrichtung (30) umfasst, die mindestens eine erste und eine zweite Verankerungseinheit (31), vorzugsweise eine Vielzahl von Verankerungseinheiten, umfasst, die mittels einer vaskulären Abgabevorrichtung, wie einem Katheter, in die Mitralklappe einsetzbar sind, und in einer Reihe entlang des Annulus positionierbar sind, umfassend Verankerungsmittel (32), die an jeder der ersten und zweiten Verankerungseinheiten (31) angeordnet sind, wobei die Verankerungseinheiten (31) so angeordnet sind, dass sie aufeinander zu bewegbar sind, so dass die Verankerungsmittel (32) in den Annulus eingreifen, wodurch der Annulus zusammengezogen wird, wenn die ersten und zweiten Verankerungseinheiten (31) bewegt werden.

## Revendications

1. Implant d'amélioration de la coaptation d'une valvule auriculo-ventriculaire dans un cœur, la valvule auriculo-ventriculaire ayant un premier feuillet natif, un second feuillet natif et éventuellement un anneau adjacent à une paroi d'un atrium du cœur, l'implant comprenant un feuillet artificiel (1) configuré pour remplacer ou supporter au moins un élément parmi les premier et second feuillets natifs, des moyens de fixation (3) pour fixer l'implant à l'anneau ou audit au moins un élément parmi les premier et second feuillets natifs et des moyens de rétention pour empêcher le prolapsus du feuillet artificiel, dans lequel les moyens de rétention comprennent ou consistent en un élément de squelette (4) s'étendant le long du feuillet artificiel (1) et vers une extrémité distale (8) du feuillet artificiel (1) et étant connecté au feuillet artificiel (1) au niveau d'au moins deux points de fixation (9, 10), dans lequel l'élément de squelette (4) est configuré pour être pliable entre un premier état de pliage et un second état de pliage, dans lequel des moyens de limitation sont prévus pour limiter le degré de flexion, et dans lequel l'élément de squelette (4) comprend une pluralité de segments (12), connectés de manière pivotante les uns aux autres, **caractérisé en ce que** les moyens de limitation comprennent un élément de butée (13) sur chaque segment (12) pour limiter l'angle de pivotement d'un segment par rapport à un segment adjacent.

2. Implant selon la revendication 1, **caractérisé en ce que** l'implant comprend un élément de repos fixé à l'extrémité proximale de l'élément de squelette (4) et configuré pour reposer contre la paroi de l'atrium adjacent à la valve.

3. Implant selon la revendication 2, **caractérisé en ce que** l'élément de repos comprend un élément de support (2) étant aligné avec l'élément de squelette (4) et configuré pour reposer contre la paroi de l'atrium adjacent à la valve.

4. Implant selon l'une quelconque des revendications 1, 2 ou 3, **caractérisé en ce que** le feuillet artificiel (1) comprend une maille (6) ou un filet flexible ou une pluralité de fils flexibles ou de fils ou un tissu ou une feuille, de préférence une feuille ou une matrice en PTFE.

5. Implant selon l'une quelconque des revendications 1 à 4, **caractérisé en ce que** le feuillet artificiel (1) comprend une armature métallique (7) s'étendant le long d'une périphérie du feuillet artificiel (1), qui est de préférence reliée à au moins deux nervures (5) s'étendant perpendiculairement à partir de l'élément de squelette (4).

6. Implant selon l'une quelconque des revendications 1 à 5, **caractérisé en ce que** le côté du feuillet artificiel (1), qui est tourné vers l'atrium, présente une surface anticoagulante et le côté du feuillet artificiel (1), qui est tourné vers le premier ou le seconde feuillet natif, présente une surface poreuse.

7. Implant selon l'une quelconque des revendications 1 à 6, **caractérisé en ce que** l'élément de squelette (4) est recouvert par une gaine.

8. Implant selon l'une quelconque des revendications 1 à 7, **caractérisé en ce qu'**une section d'extrémité distale (8) de l'élément de squelette (4) comprend un coude en U de manière à serrer une région distale du premier ou du seconde feuillet natif entre des segments de l'élément de squelette (4).

9. Implant selon la revendication 8, **caractérisé en ce que** des moyens de ressort sont agencés entre des segments (12) agencés dans la section d'extrémité distale (8) de l'élément de squelette (4), afin de presser de manière élastique des segments (12) de l'élément de squelette (4) contre la région distale du premier ou du second feuillet natif.

10. Implant selon l'une quelconque des revendications 1 à 9, **caractérisé en ce que** le moyen de fixation (3) est agencé dans la région d'extrémité proximale (11) du feuillet artificiel (1), et est de préférence aligné avec l'élément de squelette (4), et comprend une unité d'aiguille centrale (14) configurée pour pénétrer l'anneau ou ledit au moins un élément parmi les premier et second feuillets natifs dans une région de pénétration, et une plateforme de stabilisation (15) ayant une surface de butée (16) pour venir en butée contre la région de pénétration, moyennant quoi l'unité d'aiguille centrale (14) et la plateforme de stabilisation (15) sont agencées de manière mobile dans un logement (17) dans une direction axiale de l'aiguille.

11. Implant selon la revendication 10, **caractérisé en ce que** le moyen de fixation (3) comprend des premiers moyens de ressort (19) agissant sur l'unité d'aiguille (14) pour déplacer l'unité d'aiguille (14) par rapport au logement (17) et des seconds moyens de ressort (20) agissant sur la plateforme de stabilisation (15) pour déplacer la plateforme de stabilisation (15) par rapport au logement (17).

12. Implant selon la revendication 11, **caractérisé en ce que** le moyen de fixation (3) comprend en outre un premier moyen de verrouillage libérable (21) pour maintenir l'unité d'aiguille centrale (14) dans une position chargée par ressort et un second moyen de verrouillage libérable (22) pour maintenir la plateforme de stabilisation (15) dans une position chargée par ressort.

13. Implant selon la revendication 10, 11 ou 12, **caractérisé en ce que** l'unité d'aiguille centrale (14) comprend des éléments d'ancrage (23) sur son extrémité distale, lesdits éléments d'ancrage (23) étant mobiles par rapport à l'unité d'aiguille centrale (14) entre une première position, où les éléments d'ancrage (23) sont positionnés contre une tige d'aiguille (25), et une seconde position, où les éléments d'ancrage (23) sont inclinés à l'écart de la tige d'aiguille (25).

14. Implant selon l'une quelconque des revendications 2 à 13, **caractérisé en ce que** le feuillet artificiel (1) et l'élément de repos sont déployables d'une première position, dans lequel le feuillet artificiel (1) et l'élément de repos sont pliés pour être agencés à l'intérieur d'un logement tubulaire d'un dispositif de mise en place, dans une seconde position, dans laquelle le feuillet artificiel (1) et l'élément de repos sont déployés.

15. Implant selon l'une quelconque des revendications 1 à 14, **caractérisé en ce que** l'implant comprend en outre un dispositif d'annuloplastie (30) comprenant au moins une première et une seconde unités d'ancrage (31), de préférence une pluralité d'unités d'ancrage, qui peuvent être déployées sur la valve mitrale au moyen d'un dispositif de délivrance vasculaire, tel qu'un cathéter, et qui peuvent être positionnées en une rangée le long de l'anneau, comprenant des moyens d'ancrage (32) agencés sur chacune des première et seconde unités d'ancrage (31), moyennant quoi les unités d'ancrage (31) sont agencées pour être mobiles l'une vers l'autre, de sorte que les moyens d'ancrage (32) s'engagent avec l'anneau, tirant ainsi l'anneau ensemble lorsque les première et seconde unités d'ancrage (31) sont déplacées.
